# EUROPEAN PATENT SPECIFICATION

(11) **EP 0 418 310 B1**
(45) Date of publication and mention of the grant of the patent: **19.06.1996**
(21) Application number: 89906959.5
(22) Date of filing: 20.04.1989
(51) Int. Cl.: C07H 15/12, A61K 38/00

(54) **GENE FOR ENCODING A HUMAN MALARIA VACCINE ANTIGEN**
GEN ZUM KODIEREN EINES ANTIGENS GEGEN MENSCHLICHEN MALARIA IMPFSTOFF
GENE CODANT UN ANTIGENE DE VACCIN CONTRE LA MALARIA CHEZ L'HOMME

(30) Priority: 02.05.1988 US 188918
(43) Date of publication of application: 27.03.1991
(73) Proprietor: THE UNITED STATES OF AMERICA as represented by the Secretary United States Department of Commerce, Springfield, Virginia 22161 (US)
(72) Inventor: MILLER, Louis, H., Chevy Chase, MD 20815 (US); KASLOW, David, C., Kensington, MD 20895-4235 (US)
(74) Representative: Smulders, Theodorus A.H.J., Ir.
(86) International application number: US8901618
(87) International publication number: WO8910936

(56) References cited:
- WO-A-87/03882
- US-A- 4 632 909
- US-A- 4 707 357
- JOURNAL EXP. MED., vol. 162, November 1985, pages 1460-1476, The Rockefeller University Press; A.N. VERMEULEN et al.: "Sequential expression of antigens on sexual stages of plasmodium falciparum assessible to transmission-blocking antibodies in the mosquito"
- NATURE, vol. 333, no. 6168, 5th May 1988, pages 74-76, London, GB; D.C. KASLOW et al.: "A vaccine candidate from the sexual stage of human malaria that contains EGF-like domains"
- VACCINE, vol. 6, no. 2, April 1988, pages 183-187, Butterworth & Co. (Publishers) Ltd, Guilford, Surrey, GB; P. PERLMANN et al.: "Malaria vaccines: immunogen selection and epitope mapping"
- BIOCHEMISTRY, vol. 25, 1986, pages 8418-8425, American Chemical Society; S. KOBAYASHI et al.: "Partial amino acid sequence of human thrombospondin as determined by analysis of cDNA clones: Homology to malarial circumsporozoite proteins"
- J. Exp. Med., Vol. 162, issued November 1985 VERMEULEN, "Sequential Expression of Antigens on Sexual Stages of Plasmodium Falciparum Accissible to Transmission-Blocking Antibodies in the Mosquito", pages 1460-1476 see the entire article.
- CHEMICAL ABSTRACTS, Volume 109, No. 19, issued 1988 (Columbus, Ohio, USA), kASLOW, "A Vaccine Candidate from the Sexul Stage of Human Malaria that Contains EGF-like Domains". see page 547, column 2, Abstract No. 168474a, Nature (London), 1988, 333(6168), 74-6 (Eng).
- CHEMICAL ABSTRACTS, Volume 110, No. 9, issued 1989 (Columbus, Ohio, USA), CARTER "Restricted or Absent Immune Responses in Human Populations to Plasmodium Falciparum Gamete Antigens that are Targets of Malaria Transmission- Blocking Antibodies", see page 468, col. 2 Abstract No. 73510 r, J. Exp. Med. 1989, 169 (1), 135-47 (Eng).
- CHEMICAL ABSTRACTS, Volume 110, No. 7, issued 1989 (Columbus, Ohio, USA), HOLDER, "Immunization against Plasmodium Falciparum with Recombinant Polypeptides Produced in Escherichia Coli", see page 502, col. 2, Abstract No. 55534X, Parasite Immunol 1988, 10 (6), 607-17 (Eng).
- J. Exp. Med., Vol. 158, issued September 1983, RENER, "Target Antigens of transmission-Blocking Immunity on Gametes of Plasmodium Falciparum", pages 976- 981, see the entire article
- The Journal of Immunology, Vol. 137, No. 3 issued 01 August 1986, CHIZZOLINI, "Antigen-Specific and MHC-Restricted Plasmodium Falciparum-Induced Human T Lymphocyte Clones", page 1022-1028, see the entire article.

## Description

### BACKGROUND OF THE INVENTION

### 1. Field of the Invention

This invention relates to genes for encoding malarial vaccine antigens. More specifically, this invention relates to a gene for encoding the Plasmodium falciparum 25 kDA ookinete surface antigen.

### 2. Description of the Background Art

Malaria continues to exact a heavy toll from mankind. Approximately 25 percent of all deaths of children in rural Africa between the ages of one to four years are caused by Malaria. This death rate continues despite the sensitivity of local parasites to chloroquine. Mosquito control is difficult in this setting. The greatest hope at present for reducing this mortality rate is a protective vaccine that reduces the incidence of the disease and death by suppressing the replication of the parasite. The major cause of malaria in humans is the parasite Plasmodium falciparum.

The value of various vaccines to combat malaria is appreciated through an understanding of the life cycle of the parasite. Infection in man begins when young malarial parasites or "sporozoites" are injected into the blood stream of a human by the mosquito. After injection the parasite localizes in liver cells. After approximately one week the parasites or "merozoites" are released into the blood stream. The entry of the parasites into the blood stream begins the "erythrocytic" phase. Each parasite enters a red blood cell in order to grow and develop. When the merozoite matures in the red blood cell, it is known as a trophozoite and schizont. A schizont is the stage when nuclear division occurs to form individual merozoites which are released to invade other red cells. After several schizogonic cycles, some parasites, instead of becoming schizonts through asexual reproduction, develop into large uninucleate parasites. These parasites undergo sexual development.

Sexual development of the malaria parasites involves the female or "macrogametocyte" and the male parasite or "microgametocyte". These gametocytes do not undergo any further development in man. Upon ingestion of the gametocytes into the mosquito, the complicated sexual cycle begins in the midgut of the mosquito. The red blood cells disintegrate in the midgut of the mosquito after 10 to 20 minutes. The microgametocyte continues to develop through exflagellation and releases 8 highly flagellated microgametes. Fertilization occurs with the fusion of the microgamete into a macrogamete. The fertilized parasite is known as a zygote that develops into an "ookinete". The ookinete penetrates the midgut wall of the mosquito and transforms into the oocyst within which many small sporozoites form. When the oocyst ruptures the sporozoites migrate to the salivary gland of the mosquito via the haemolymph. Once in the saliva of the mosquito, the parasite can be injected into a host.

Malaria vaccines are being developed against different stages in the parasite's life-cycle which includes the sporozoite, asexual erythrocyte, and sexual stage. Each development increases the opportunity to control malaria in the many diverse settings within which the disease occurs. Sporozoite vaccines would prevent mosquito-induced infections. First generation vaccines of this type have been tested in humans. Asexual erythrocytic stage vaccines would be useful in reducing the severity of the disease. Multiple candidate antigens have been cloned and tested in animals and in humans.

Sexual stage vaccines would induce antibodies which, when ingested in a bloodmeal containing sexual stage parasites, would prevent infection of mosquitoes. Although not directly protective against infection or disease, the sexual stage vaccine combined with a protective vaccine such as a sporozoite or asexual stage vaccine would reduce the chance of transmission of vaccine-induced mutants resistant to the protective component. In this manner, the useful life of the protective component would be lengthened. In some geographical areas the sexual stage vaccine could reduce transmission below the critical threshold required to maintain the infected population. This reduced transmission would be useful in assisting in the control or eradication of malaria.

U.S. Patent Number 4,632,909 to Carter and Miller, herein incorporated by reference, discloses monoclonal antibodies that bind with one or more proteins located on the surface of gametes or zygotes of malaria parasites and are targets for sexual stage vaccines. These antibodies are specific for antigens on mosquito midgut stages of malaria parasite and sterilize the parasites in mosquitoes otherwise capable of transmitting the disease. The monoclonal antibodies are specific for the 255, 59 and 53 K surface proteins on Plasmodium falciparum and for the 25 K surface protein on zygotes and ookinetes of Plasmodium gallinaceum. This invention includes a process for blocking transmissions of malaria parasites. The process involves the feeding of mosquitoes carrying the malaria parasite monoclonal antibodies specific for a glycoprotein on the surface of the malaria parasite zygote. The glycoprotein has a molecular weight of 24-30 K. The process is effective in a zygote up to about 3 hours after fertilization. This invention does not involve a cloned gene to induce transmission blocking immunity to malaria nor a deduced peptide from the gene for use in a malarial vaccine.

A study to identify antigens useful to develop malaria transmission blocking immunity is disclosed in the article, Carter et al., "Target Antigens in Malaria Transmission Blocking Immunity," Phil. Trans. R. Soc. Land. B 307:201-213 (1984), herein incorporated by reference. This article describes the phases of development of malaria parasites wherein transmission blocking immunity occurs. Target antigens on gametes and newly fertilized zygotes and target antigens of post-fertilization transmission blocking immunity are identified in the article. This article does not disclose a cloned gene to induce transmission blocking immunity to malaria nor a deduced peptide from the gene for use in a malarial vaccine.

An article, Grotendorst et al., "A Surface Protein Expressed During the Transformation of Zygotes of Plasmodium gallinaceum is a Target of Transmission-Blocking Antibodies," Infection and Immunity, Vol. 45, No. 3, p. 775-777 (1984), herein incorporated by reference, discloses a specific protein suitable for use as an antigen. This article identifies materials and procedures that are useful in isolating and identifying an antigen which is a surface protein of Mᵣ 26,000 synthesized by zygotes of P. gallinaceum. Monoclonal antibodies, having properties of anti-ookinete serum, were found in certain examples to suppress infectivity of fertilized parasites to mosquitoes. An analogous 25kDa surface protein synthesized by zygotes and ookinetes of Plasmodium falciparum is described by Vermeulen et al., "Sequential Expression of Antigens on Sexual Stages of Plasmodium Falciparum accessible to Transmission-blocking Antibodies in the Mosquito," J. Exp. Med. 162:1460-1476 (1985), herein incorporated by reference. These articles do not disclose a cloned gene of P. falciparum to induce transmission blocking immunity to malaria nor a deduced peptide from the gene for use in a malarial vaccine.

The industry is lacking a gene which can produce a vaccine designed to induce transmission blocking immunity to Plasmodium falciparum 25kDa surface protein (herein after Pfs25) and other sexual stage antigens. The industry is also lacking a synthetic peptide that can be expressed from the above gene and used in a pharmaceutical composition to produce a malarial vaccine. Vaccines derived from such genes would prolong the usefulness of other protective malarial vaccines as well as reduce the spread of malaria in areas of low transmission.

### SUMMARY OF THE INVENTION

The invention is a gene for expressing antigens for producing a human malaria vaccine. The gene includes a cloned nucleotide sequence or segment for encoding the 25kDa surface protein of zygotes and ookinetes of Plasmodium falciparum. The segment of the gene encoding the protein is

ATG AAT AAA CTT TAC AGT TTG TTT CTT TTC CTT TTC ATT CAA CTT
AGC ATA AAA TAT AAT AAT GCG AAA GTT ACC GTG GAT ACT GTA TGC
AAA AGA GGA TTT TTA ATT CAG ATG AGT GGT CAT TTG GAA TGT AAA
TGT GAA AAT GAT TTG GTG TTA GTA AAT GAA GAA ACA TGT GAA GAA
AAA GTT CTG AAA TGT GAC GAA AAG ACT GTA AAT AAA CCA TGT GGA
GAT TTT TCC AAA TGT ATT AAA ATA GAT GGA AAT CCC GTT TCA TAC
GCT TGT AAA TGT AAT CTT GGA TAT GAT ATG GTA AAT AAT GTT TGT
ATA CCA AAT GAA TGT AAG AAT GTA ACT TGT GGT AAC GGT AAA TGT
ATA TTA GAT ACA AGC AAT CCT GTT AAA ACT GGA GTT TGC TCA TGT
AAT ATA GGC AAA GTT CCC AAT GTA CAA GAT CAA AAT AAA TGT TCA
AAA GAT GGA GAA ACC AAA TGC TCA TTA AAA TGC TTA AAA GAA AAT
GAA ACC TGT AAA GCT GTT GAT GGA ATT TAT AAA TGT GAT TGT AAA
GAT GGA TTT ATA ATA GAT AAT GAA AGC TCT ATA TGT ACT GCT TTT
TCA GCA TAT AAT ATT TTA AAT CTA AGC ATT ATG TTT ATA CTA TTT
TCA GTA TGC TTT TTT ATA ATG TAA.

The invention includes a synthetic protein which is useful for preparing a malaria vaccine. The synthetic protein of the cloned gene is

Met Asn Lys Leu Tyr Ser Leu Phe Leu Phe Leu Phe Ile Gln Leu
Ser Ile Lys Tyr Asn Asn Ala Lys Val Thr Val Asp Thr Val Cys
Lys Arg Gly Phe Leu Ile Gln Met Ser Gly His Leu Glu Cys Lys
Cys Glu Asn Asp Leu Val Leu Val Asn Glu Glu Thr Cys Glu Glu

Lys Val Leu Lys Cys Asp Glu Lys Thr Val Asn Lys Pro Cys Gly
Asp Phe Ser Lys Cys Ile Lys Ile Asp Gly Asn Pro Val Ser Tyr
Ala Cys Lys Cys Asn Leu Gly Tyr Asp Met Val Asn Asn Val Cys
Ile Pro Asn Glu Cys Lys Asn Val Thr Cys Gly Asn Gly Lys Cys
Ile Leu Asp Thr Ser Asn Pro Val Lys Thr Gly Val Cys Ser Cys
Asn Ile Gly Lys Val Pro Asn Val Gln Asp Gln Asn Lys Cys Ser
Lys Asp Gly Glu Thr Lys Cys Ser Leu Lys Cys Leu Lys Glu Asn
Glu Thr Cys Lys Ala Val Asp Gly Ile Tyr Lys Cys Asp Cys Lys
Asp Gly Phe Ile Ile Asp Asn Glu Ser Ser Ile Cys Thr Ala Phe
Ser Ala Tyr Asn Ile Leu Asn Leu Ser Ile Met Phe Ile Leu Phe
Ser Val Cys Phe Phe Ile Met.

The invention includes a pharmaceutical composition having the synthetic protein and the method to make an anti-malarial vaccine including the synthetic protein.

### BRIEF DESCRIPTION OF THE DRAWINGS

Figure 1 illustrates the nucleotide and predicted amino acid sequence of Pfs25.

Figure 2 illustrates a Northern blot analysis of asexual and sexual stage RNA.

Figure 3 illustrates a protein structure of Pfs25 arranged to emphasize the relatedness of the EGF-like domains.

### DETAILED DESCRIPTION OF THE INVENTION

The invention is the isolated and cloned gene for encoding the 25 kDa surface protein (Pfs25) of zygotes and ookinetes of Plasmodium falciparum. The deduced amino acid sequence of this gene consists of a signal sequence, a hydrophobic C-terminus, and four tandem epidermal growth factor (EGF)-like domains. The cloned gene of this invention, therefore, provides a useful composition that can express an antigen that is useful in preparing a malarial vaccine. The antigen is also a useful product of this invention. The antigen is a polypeptide that can be used in therapeutic quantities to prepare pharmaceutical compositions which are suitable as malaria vaccines.

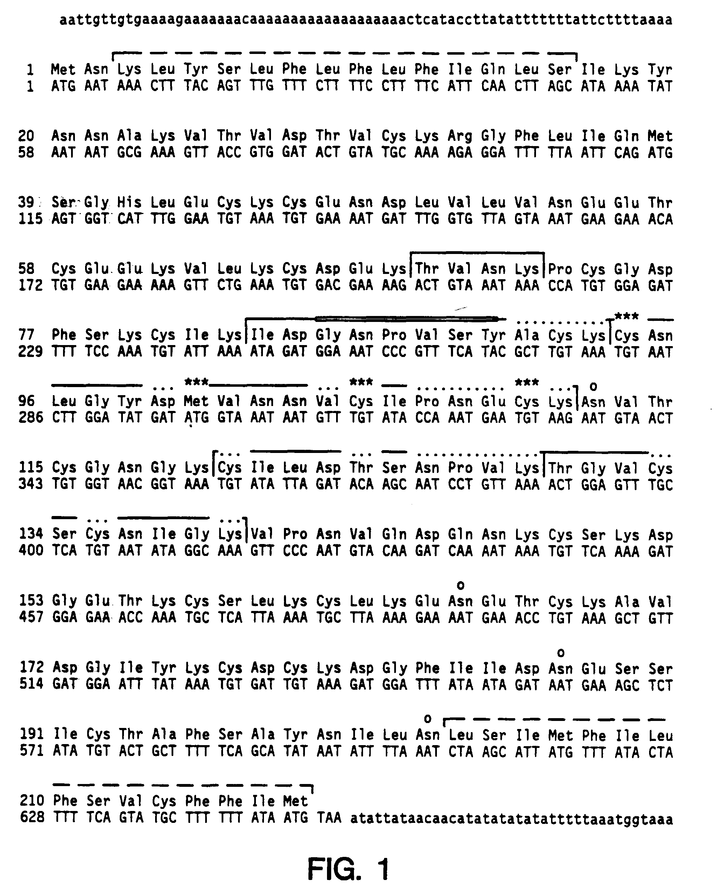

The genes for encoding the three sexual stage-specific antigens of P. falciparum have not been cloned to date. This is in part due to the fact that monoclonal antibody defined epitopes are dependent on disulfide bonds and large quantities of parasites and purified protein needed for peptide sequencing are difficult to obtain. The gene of this invention is obtained by purifying Pfs25 from zygotes of P. falciparum. In the preferred embodiment of the invention the 3D7 clone of NF54 P. falciparum is used. The Pfs25 is purified by using immunoaffinity chromatography and SDS-PAGE. The microsequence of the protein is then performed in order to make oligonucleotide probes to screen genomic DNA libraries. The purified Pfs25 is digested with trypsin, because the amino-terminus was blocked. The resulting peptides are fractionated by HPLC. Five peptide sequences are obtained from this process. These peptide sequences are identified in Figure 1.

A highly degenerate oligonucleotide probe is constructed from tryptic peptide sequence. This oligonucleotide is used to clone a 600 bp Dra I fragment (pSKR 2) of genomic DNA. The Dra I fragment contains one long open reading frame but no termination codon. Therefore, a 3.5 kb Hind III fragment (pNF4.13) is cloned. Both cloned fragments can be used to determine the nucleotide sequence of Pfs25.

Figure 1 illustrates the amino acid sequence for Pfs25 as deduced by the above process. This amino acid sequence is shown above the nucleotide sequence. The gene has a single exon that codes for a polypeptide of 217 amino acids. The predicted coding region of the nucleotide sequence is capitalized and consists of 654 base pairs. The N-terminus is blocked; therefore, the start of the mature protein has not been determined. Solid lines represent tryptic peptide sequences determined by microsequencing. Asterisks represent the sequence determined by microsequencing radiolabelled peptides. The dotted line represents the indeterminate amino acid residue of the microsequenced peptide. The double solid line represents the tryptic peptide sequence used to construct oligonucleotide probes. The open circles represent the sites of possible asparagine-linked glycosylation. The broken lines represent the hydrophobic regions. First and most important in providing evidence that the gene for the the 25kDa ookinete surface antigen is cloned is that five of the microsequenced tryptic peptides of Pfs25 are found within the deduced amino acid sequence of Pfs25 as shown in Figure 1. A preparation of (³⁵S)-labelled Pfs25 can be immunopurified from zygotes that had been metabolically labelled with ³⁵S-methionine and ³⁵S-cysteine. The resulting tryptic peptides of Pfs25 are separated by HPLC and the fraction containing the most radioactivity is microsequenced. The radioactive peaks for cysteine and methionine in the tryptic peptide of Pfs25 perfectly match the position of these residues in the deduced amino acid sequence of the Pfs25 gene of Figure 1 marked by asterisks.

A malarial vaccine can be produced from the gene of Figure 1 or a portion thereof. The gene can be modified using known techniques to delete the signal sequence, the hydrophobic anchor, and/or other portions of the gene. These modifications to the gene continue to produce a suitable antigen for use in the vaccine.

Those skilled in the art can understand that certain codons presented in the sequence of Figure 1 can be substituted by other codons and produce an equivalent segment. The polypeptide produced by the equivalent segment has a corresponding change in its amino acid sequence, but has an equivalent function to the preferred polypeptide. For example, a substitution of glutamic acid for glycine at amino acid 131 does not alter the function of the polypeptide.

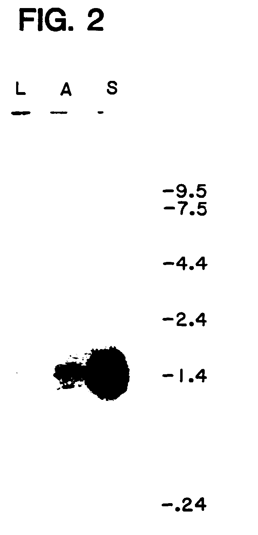

Second, the gene of this invention is expressed preferentially in the sexual stages of P. falciparum. This is the stage in which Pfs25 is synthesized. The mRNA from gametocytes (not shown) and five hour old zygotes as illustrated in Figure 2, lane 5, of P. falciparum using the 3D7 clone of NF 54, have an abundance of an approximately 1.4 kb species that hybridizes to pSKR 2. In contrast, the mRNA from asexual stage parasites of 3D7 as illustrated in Figure 2, lane A, gives only a weak signal. The weak signal from the asexual stage parasites is most likely due to the presence of some gametocytes in the preparation of parasites. For example pSKR 2 does not hybridize with the mRNA as illustrated in Figure 2, lane L, from a P. falciparum parasite that produces no gametocytes such as the LF4 clone of a Liberian isolate of P. falciparum.

Third, when gametes, zygotes, or ookinetes of P. falciparum are metabolically labeled, multiple sexual stage proteins incorporate ³⁵S-methionine and Pfs25 is not a major radiolabelled product. In contrast, Pfs25 is the predominate product incorporating ³⁵S-cysteine as known by tests which are standard in the art. The deduced amino acid sequence contains 11 percent cysteine which correlates with these results.

Fourth, the structure of the protein from the deduced amino acid sequence is consistent with previous reports as cited above of the biochemistry of the 25 kDa surface glycoprotein. The deduced sequence contains a putative signal peptide at the N-terminus and a short hydrophobic anchor at the C-terminus. It has four potential glycosylation sites for N-linked sugars and encodes for a polypeptide of approximately 24 kDa. The deduced sequence also has a short hydrophobic anchor of 15 amino acids and the lack of a potential cytoplasmic hydrophilic region at the C-terminus.

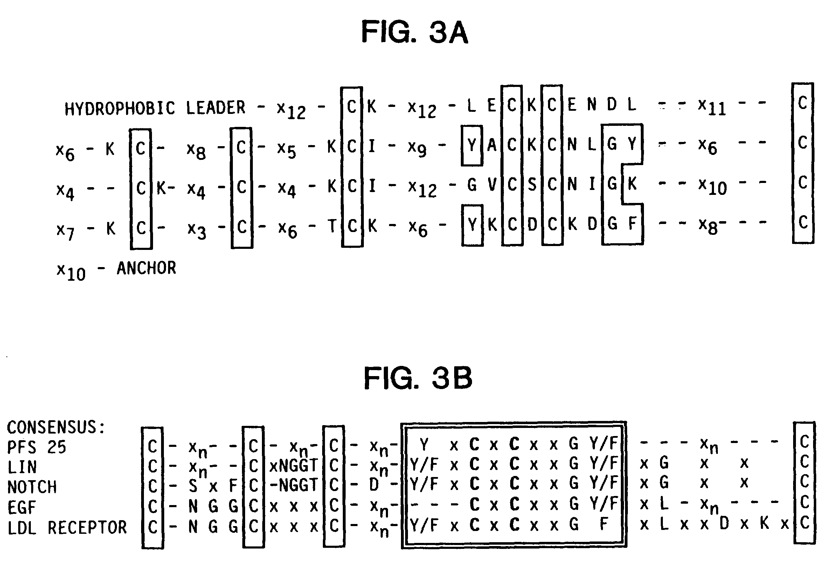

The organization of the cysteines between the signal sequence and the hydrophobic C-terminus is similar to the domains in EGF 1 as illustrated in Figure 3B. Based on the position of the cysteines in the exons of human EGF precursor and human LDL receptor, three cysteine residues precede the consensus sequence Y/F-x-C x-C x-x-G-Y/F and one follows it as illustrated in Figure 3B. The EGF-like domain is also found in invertebrates such as notch in Drosophilia melanogaster and lin-12 in Caenorhabditis elegans. This invention provides the first report of the presence of EGF-like domains in proteins of unicellular organisms. The presence of EGF-like domains in the protein of this invention can be expected to have a growth factor effect on higher organisms including the mosquito.

### EXAMPLE

The following example provides the procedure for obtaining the gene of the invention. This example represents the preferred embodiment of the invention.

A 3D7 clone of an NF 54 isolate of P. falciparum was cultured in vitro and zygotes were prepared as described above. Pfs25 from Triton X-100 extracts of five hour old zygotes (10⁹) was immunoaffinity purified using monoclonal antibody 1C7 covalently linked to Sepharose 4B beads (CnBr-activated). Pfs25 had been metabolically labeled with trans S35 which has 70% ³⁵S-methionine, 20% ³⁵S-cysteine, and 10% other ³⁵S-compounds and is commercially available from ICN Radiochemicals, Inc. The beads contained Pfs25 and were resuspended in SDS-PAGE sample buffer having 5% SDS, 62.5mM Tris at pH 6.8, 0.002% Bromophenol blue, and 8 M urea. These were heated at 68°C for five minutes. The eluted protein in the sample buffer was loaded onto a 12% SDS-polyacrylamide gel under nonreducing conditions. The Pfs25 was the only radiolabelled protein identified on the gel and was recovered from the gel by passive diffusion. The lyophilized sample was resolubilized in 0.5 M Tris-HCl pH 8.5, 6 M guanidine hydrochloride, 0.3 mM EDTA buffer containing 64 mM dithiothreitol, and incubated for 2 hours at 37°C in a N₂ atmosphere. Iodoacetamide was added to a final concentration of 174 mM and reacted for 1 hour at 25°C in the dark. An excess of 2-mercaptoethanol was added followed by 10 volumes of absolute ethanol. The reduced and alkylated protein was allowed to precipitate at -20°C for 4 hours. The remaining pellet was resuspended in 50 mM NH₄HCO₃, pH 7.9 and digested with two 0.5 ug doses of TPCK treated trypsin (Sigma), each dose being followed by a 6 hour incubation at 37°C. The digestion was terminated by heating the sample for 10 minutes at 65°C. The tryptic peptides were fractionated on a reverse phase HPLC (RP-300, Applied Biosystems, Inc.) using an 50% acetonitrile gradient with 0.1% trifluoracetic acid. Peptide microsequencing was performed on a model 470A gas phase sequencer from Applied Biosystems, Inc. 40% of each cycle's product was analyzed on an attached model 120A PTH analyzer using the manufacturer's program, C3RPTH. For one peptide, the radioactivity of the remaining 60% of each cycle's product was determined. Radioactive peaks (***) were found in cycle numbers 1, 7, 12, and 17.

The peptide sequence identified by double solid lines was used to construct highly degenerate oligonucleotide probes. A group of oligonucleotides of 512 degeneracy hybridized to a 1.4 kb band of zygote RNA. By varying the codon for proline, the probe was divided into four groups of 128 degeneracy each, one group of which hybridized to a 1.4 kb band of RNA as well as a 600 bp Dra I fragment of genomic DNA. 20,000 plaques, of a size-selected Dra I fragment library in lambda gt 10, were screened with this oligonucleotide probe. One clone was identified and subcloned into Blue-script SK (pSKR 2). pSKR 2 was used to identify a 3.5 kb Hind III fragment in a size selected library in the vector pSP64. Both strands of each clone were sequenced by the dideoxynucleotide terminator method.

In Figure 2 the Northern blot analysis of asexual and sexual stage RNA is shown. Total cellular RNA (20 ug/lane) was prepared from asexual parasites (L) of LF 4, asexual parasites (A) of 3D7, or 5 hour old zygotes (S) of 3D7 were electrophoresed through a 1% agarose/formaldehyde gel and transferred to a nylon membrane. The filter was hybridized at 55°C overnight with randomly primed pSKR 2 insert (specific activity 10⁹ c.p.m. ug⁻¹) and washed as described Size markers are 0.24-9.5 kb RNA ladder (BRL).

Figure 3 illustrates in part A protein structure of Pfs25 arranged to emphasize the relatedness of the EGF-like domains. The boxes represent cysteine residues and other identical or related amino acids. Part B illustrates EGF-like repeat consensus sequence from Pfs25, lin-12, notch, EGF, and human LDL receptor. The boxes represent cysteine residues. The double box represents a core of consensus sequence. The letters designate the following amino acids: C, Cys; D, Asp; E, Glu; F, Phe; G, Gly; I, Ile; K; Lys; L, Leu; N, Asn; T, Thr; Y, Tyr; x, any amino acid; and unspecified number of amino acids.

## Claims

1. A recombinant DNA molecule encoding a 25 kDa surface protein or an antigenic portion thereof of zygotes and ookinetes of Plasmodium falciparum having a signal sequence, a hydrophobic C-terminus and four tandem epidermal growth factor-like domains.

2. A recombinant DNA molecule encoding a 25 kDa surface protein or an antigenic portion thereof of zygotes and ookinetes of Plasmodium falciparum having a signal sequence, a hydrophobic C-terminus and four tandem epidermal growth factor-like domains for expressing an antigen for producing a human malaria vaccine.

3. A recombinant DNA molecule encoding the amino acid sequence: 
Met Asn Lys Leu Tyr Ser Leu Phe Leu Phe Leu Phe Ile Gln Leu
Ser Ile Lys Tyr Asn Asn Ala Lys Val Thr Val Asp Thr Val Cys
Lys Arg Gly Phe Leu Ile Gln Met Ser Gly His Leu Glu Cys Lys
Cys Glu Asn Asp Leu Val Leu Val Asn Glu Glu Thr Cys Glu Glu
Lys Val Leu Lys Cys Asp Glu Lys Thr Val Asn Lys Pro Cys Gly
Asp Phe Ser Lys Cys Ile Lys Ile Asp Gly Asn Pro Val Ser Tyr
Ala Cys Lys Cys Asn Leu Gly Tyr Asp Met Val Asn Asn Val Cys
Ile Pro Asn Glu Cys Lys Asn Val Thr Cys Gly Asn Gly Lys Cys
Ile Leu Asp Thr Ser Asn Pro Val Lys Thr Gly Val Cys Ser Cys
Asn Ile Gly Lys Val Pro Asn Val Gln Asp Gln Asn Lys Cys Ser
Lys Asp Gly Glu Thr Lys Cys Ser Leu Lys Cys Leu Lys Glu Asn
 
Glu Thr Cys Lys Ala Val Asp Gly Ile Tyr Lys Cys Asp Cys Lys
Asp Gly Phe Ile Ile Asp Asn Glu Ser Ser Ile Cys Thr Ala Phe
Ser Ala Tyr Asn Ile Leu Asn Leu Ser Ile Met Phe Ile Leu Phe
Ser Val Cys Phe Phe Ile Met
 or an antigenic portion thereof.

4. A recombinant DNA molecule comprising the following DNA sequence:

ATG AAT AAA CTT TAC AGT TTG TTT CTT TTC CTT TTC ATT CAA CTT
AGC ATA AAA TAT AAT AAT GCG AAA GTT ACC GTG GAT ACT GTA TGC
AAA AGA GGA TTT TTA ATT CAG ATG AGT GGT CAT TTG GAA TGT AAA
TGT GAA AAT GAT TTG GTG TTA GTA AAT GAA GAA ACA TGT GAA GAA
AAA GTT CTG AAA TGT GAC GAA AAG ACT GTA AAT AAA CCA TGT GGA
GAT TTT TCC AAA TGT ATT AAA ATA GAT GGA AAT CCC GTT TCA TAC
GCT TGT AAA TGT AAT CTT GGA TAT GAT ATG GTA AAT AAT GTT TGT
ATA CCA AAT GAA TGT AAG AAT GTA ACT TGT GGT AAC GGT AAA TGT
ATA TTA GAT ACA AGC AAT CCT GTT AAA ACT GGA GTT TGC TCA TGT
AAT ATA GGC AAA GTT CCC AAT GTA CAA GAT CAA AAT AAA TGT TCA
AAA GAT GGA GAA ACC AAA TGC TCA TTA AAA TGC TTA AAA GAA AAT
GAA ACC TGT AAA GCT GTT GAT GGA ATT TAT AAA TGT GAT TGT AAA
GAT GGA TTT ATA ATA GAT AAT GAA AGC TCT ATA TGT ACT GCT TTT
TCA GCA TAT AAT ATT TTA AAT CTA AGC ATT ATG TTT ATA CTA TTT
TCA GTA TGC TTT TTT ATA ATG TAA.

## Patentansprüche

1. Rekombinantes DNA-Molekül, das ein 25 kDA-Oberfläche-Protein oder ein antigenes Teil davon von Zygoten und Ookineten von Plasmodium falciparum verschlüsselt, mit einer Signalfrequenz, einem hydrophoben C-Terminus und vier hintereinander liegenden, epidermalen wachstumsfaktorähnlichen Bereichen.

2. Rekombinantes DNA-Molekül, das ein 25 kDA-Oberfläche-Protein oder ein antigenes Teil davon in Zygoten und Ookineten von Plasmodium falciparum verschlüsselt, mit einer Signalfrequenz, einem hydrophoben C-Terminus und vier epidermalen wachstumsfaktorähnlichen Bereichen zum Ausdrücken eines Antigens zur Erzeugung eines menschlichen Malaria-Impfstoffs.

3. Rekombinantes DNA-Molekül, das folgende Aminosäure-Sequenz verschlüsselt: 
Met Asn Lys Leu Tyr Ser Leu Phe Leu Phe Leu Phe Ile Gln Leu
Ser Ile Lys Tyr Asn Asn Ala Lys Val Thr Val Asp Thr Val Cys
Lys Arg Gly Phe Leu Ile Gln Met Ser Gly His Leu Glu Cys Lys
Cys Glu Asn Asp Leu Val Leu Val Asn Glu Glu Thr Cys Glu Glu
Lys Val Leu Lys Cys Asp Glu Lys Thr Val Asn Lys Pro Cys Gly
Asp Phe Ser Lys Cys Ile Lys Ile Asp Gly Asn Pro Val Ser Tyr
Ala Cys Lys Cys Asn Leu Gly Tyr Asp Met Val Asn Asn Val Cys
Ile Pro Asn Glu Cys Lys Asn Val Thr Cys Gly Asn Gly Lys Cys
Ile Leu Asp Thr Ser Asn Pro Val Lys Thr Gly Val Cys Ser Cys
Asn Ile Gly Lys Val Pro Asn Val Gln Asp Gln Asn Lys Cys Ser
Lys Asp Gly Glu Thr Lys Cys Ser Leu Lys Cys Leu Lys Glu Asn
 
Glu Thr Cys Lys Ala Val Asp Gly Ile Tyr Lys Cys Asp Cys Lys
Asp Gly Phe Ile Ile Asp Asn Glu Ser Ser Ile Cys Thr Ala Phe
Ser Ala Tyr Asn Ile Leu Asn Leu Ser Ile Met Phe Ile Leu Phe
Ser Val Cys Phe Phe Ile Met
 oder ein Antigen-Teil davon.

4. Rekombinantes DNA-Molekül, das die folgende DNA-Sequenz umfaßt:

ATG AAT AAA CTT TAC AGT TTG TTT CTT TTC CTT TTC ATT CAA CTT
AGC ATA AAA TAT AAT AAT GCG AAA GTT ACC GTG GAT ACT GTA TGC
AAA AGA GGA TTT TTA ATT CAG ATG AGT GGT CAT TTG GAA TGT AAA
TGT GAA AAT GAT TTG GTG TTA GTA AAT GAA GAA ACA TGT GAA GAA
AAA GTT CTG AAA TGT GAC GAA AAG ACT GTA AAT AAA CCA TGT GGA
GAT TTT TCC AAA TGT ATT AAA ATA GAT GGA AAT CCC GTT TCA TAC
GCT TGT AAA TGT AAT CTT GGA TAT GAT ATG GTA AAT AAT GTT TGT
ATA CCA AAT GAA TGT AAG AAT GTA ACT TGT GGT AAC GGT AAA TGT
ATA TTA GAT ACA AGC AAT CCT GTT AAA ACT GGA GTT TGC TCA TGT
AAT ATA GGC AAA GTT CCC AAT GTA CAA GAT CAA AAT AAA TGT TCA
AAA GAT GGA GAA ACC AAA TGC TCA TTA AAA TGC TTA AAA GAA AAT
GAA ACC TGT AAA GCT GTT GAT GGA ATT TAT AAA TGT GAT TGT AAA
GAT GGA TTT ATA ATA GAT AAT GAA AGC TCT ATA TGT ACT GCT TTT
TCA GCA TAT AAT ATT TTA AAT CTA AGC ATT ATG TTT ATA CTA TTT
TCA GTA TGC TTT TTT ATA ATG TAA.

## Revendications

1. Molécule d'ADN recombiné codant pour une protéine de surface de 25 kDa ou une de ses portions antigéniques de zygotes et d'oocinètes de *Plasmodium falciparum* ayant une séquence-signal, une extrémité C-terminale hydrophobe et quatre domaines en tandem semblables au facteur de croissance épidermique.

2. Molécule d'ADN recombiné codant pour une protéine de surface de 25 kDa ou une de ses portions antigéniques de zygotes et d'oocinètes de *Plasmodium falciparum* ayant une séquence-signal, une extrémité C-terminale hydrophobe et quatre domaines en tandem semblables au facteur de croissance épidermique, pour exprimer un antigène destiné à produire un vaccin antimalarique pour l'homme.

3. Molécule d'ADN recombiné codant pour la séquence d'amino-acides : 
Met Asn Lys Leu Tyr Ser Leu Phe Leu Phe Leu Phe Ile Gln Leu
Ser Ile Lys Tyr Asn Asn Ala Lys Val Thr Val Asp Thr Val Cys
Lys Arg Gly Phe Leu Ile Gln Met Ser Gly His Leu Glu Cys Lys
Cys Glu Asn Asp Leu Val Leu Val Asn Glu Glu Thr Cys Glu Glu
Lys Val Leu Lys Cys Asp Glu Lys Thr Val Asn Lys Pro Cys Gly
Asp Phe Ser Lys Cys Ile Lys Ile Asp Gly Asn Pro Val Ser Tyr
Ala Cys Lys Cys Asn Leu Gly Tyr Asp Met Val Asn Asn Val Cys
Ile Pro Asn Glu Cys Lys Asn Val Thr Cys Gly Asn Gly Lys Cys
Ile Leu Asp Thr Ser Asn Pro Val Lys Thr Gly Val Cys Ser Cys
Asn Ile Gly Lys Val Pro Asn Val Gln Asp Gln Asn Lys Cys Ser
Lys Asp Gly Glu Thr Lys Cys Ser Leu Lys Cys Leu Lys Glu Asn
Glu Thr Cys Lys Ala Val Asp Gly Ile Tyr Lys Cys Asp Cys Lys
Asp Gly Phe Ile Ile Asp Asn Glu Ser Ser Ile Cys Thr Ala Phe
Ser Ala Tyr Asn Ile Leu Asn Leu Ser Ile Met Phe Ile Leu Phe
Ser Val Cys Phe Phe Ile Met
 ou une portion antigénique de celle-ci.

4. Molécule d'ADN recombiné comprenant la séquence suivante d'ADN :
